# FASCICULE DE BREVET EUROPEEN

(11) **EP 1 351 680 B1**
(45) Date de publication et mention de la délivrance du brevet: **15.06.2005**
(21) Numéro de dépôt: 01929703.5
(22) Date de dépôt: 25.04.2001
(51) Int. Cl.: A61K 31/385, A61P 31/00

(54) **UTILISATION DE L'ANETHOLE-DITHIOLETHIONE DANS LA PREVENTION ET LE TRAITEMENT DE LA TENOXICITE INDUITE PAR UN MEDICAMENT**
VERWENDUNG VON ANETHOL-DITHIOLETHION ZUR VORBEUGUNG UND BEHANDLUNG DER SEHNENTOXIZITÄT
USE OF ANETHOLE-DITHIOLETHIONE FOR PREVENTING AND TREATING DRUG-INDUCED TENDON TOXICITY

(30) Priorité: 25.04.2000 FR 0005247
(43) Date de publication de la demande: 15.10.2003
(73) Titulaire: Solvay Pharma SAS, 92150 Suresnes (FR)
(72) Inventeur: CHRISTEN, Marie-Odile, F-75116 Paris (FR); WARNET, Jean-Michel, F-75016 Paris (FR); RAT, Patrice, F-75012 Paris (FR)
(74) Mandataire: Bernasconi, Jean
(86) Numéro de dépôt international: PCT/FR2001/001268
(87) Numéro de publication internationale: WO 2001/080856

(56) Documents cités:
- SIMONIN MARIE-AGNES ET AL: "Pefloxacin-induced Achilles tendon toxicity in rodents: Biochemical changes in proteoglycan synthesis and oxidative damage to collagen." ANTIMICROBIAL AGENTS AND CHEMOTHERAPY, vol. 44, no. 4, avril 2000 (2000-04), pages 867-872, XP000989664 ISSN: 0066-4804
- CHRISTEN MARIE-ODILE: "Anethole dithiolethione research overview and perspectives." PROCEEDINGS OF THE INTERNATIONAL SYMPOSIUM ON NATURAL ANTIOXIDANTS:, 1996, pages 236-242, XP000989749 Meeting;Beijing, China; June 20-24, 1995, 1996 AOCS Press Champaign, Illinois, USA ISBN: 0-935315-69-1
- CHRISTEN M O: "Anethole dithiolethione: Overview on its biochemical properties and perspectives" NAUNYN-SCHMIEDEBERGS ARCHIVES OF PHARMACOLOGY, (SEP 1998) VOL. 358, NO. 1, SUPP. [1], PP. P4045-P4045. PUBLISHER: SPRINGER VERLAG, 175 FIFTH AVE, NEW YORK, NY 10010. ISSN: 0028-1298., XP000989709 SOLVAY PHARMA, F-92151 SURESNES, FRANCE

## Description

La présente invention concerne une nouvelle utilisation de l'anéthole-dithiolethione (ou anéthole trithione).

Les quinolones et notamment les quinolones de deuxième génération, les fluoroquinolones sont des antibiotiques de synthèse qui, en raison de leur très grande activité, sont très largement utilisés.

Ces produits sont relativement bien tolérés ; néanmoins un effet secondaire a été relevé depuis les années 90. Il s'agit de tendinopathies, regroupant tendinites et ruptures tendineuses apparaissant dans la majorité des cas au niveau du tendon d'Achille, mais pouvant également toucher le tendon quadricipital, ou, au niveau de l'épaule, le tendon du sus-épineux. La durée d'invalidité est en moyenne de 62 jours pour des tendinites simples et 98 jours pour des ruptures. (Simonin et al., Antimicrobial agents and chemo therapy, 44 (4), April 2000, 867-872)

D'autres molécules semblent avoir des effets similaires, telles , les statines. En effet, certains cas de tendinopathies et de ruptures de tendon ont été répertoriés avec la simvastatine (Zocor® ou Lodales®) mais aussi avec les nouvelles générations de statines récement commercialisées.

Ce phénomène de ténotoxicité était jusqu'à présent mal connu et les moyens ou produits pour y remédier inexistants.

La cellule constitutive du tendon est le ténocyte ; dans le tendon, ces cellules sont organisées en colonnes entre les fibres de collagène.
Les auteurs de la présente invention ont étudié dans un modèle cellulaire de ténocyte l'effet cytotoxique des fluoroquinolones. Ils ont ensuite constaté dans ce modèle que l'anéthole-dithiolethione (Sulfarlem®), testé en prétraitement avant mise en contact avec ces xénobiotiques, protège de façon surprenante et unique contre cette ténotoxicité, montrant l'intérêt de cette molécule dans la prévention et le traitement de la ténotoxicité induite par des médicaments tels que les quinolones ou les statines.

L'anéthole-dithiolethione peut donc être utilisée en tant que médicament administré seul ou en coadministration avec l'antibiotique ou la statine en prévention de la ténotoxicité induite par des xénobiotiques tels que les quinolones ou les statines.

La présente invention a donc pour objet l'utilisation de l'anéthole-dithiolelhione pour la préparation d'un médicament destiné à la prévention et au traitement de la ténotoxicité induite par des médicaments tels que les quinolones ou les statines, contenant l'anéthole-dithiolethione seule ou d'un mélange ou d'une association avec un médicament inducteur.

L' anéthole-dithiolethione peut être utilisée sous une forme contenant de 10 à 200 mg et peut être administrée à des doses de 10 à 200 mg/jour.

On donnera ci-après des résultats d'essais mettant en évidence l'effet de protection de l'anéthole-dithiolethione.

Le test effectué est un test MIFALC (Microtitration Fluorimetric Assay on Living Cells) de type II , c'est à dire que toutes les étapes y-compris la détection / révélation , se font sur cellules vivantes, le test utilisant une détection fluorimétrique en lumière froide, afin d'obtenir la sensibilité et la spécificité de détection maximale nécessaire aux études intracellulaires.Les essais sont effectués sur une lignée de ténocytes immortalisés, appelés TENO (Teno Cell Line).
■ *Préparation des cultures*
   Les cultures sont réalisées en flacons de 75 cm³ pour la multiplication cellulaire et en plaques de 96 puits à fond plat pour culture.
   Les ténocytes sont mis en suspension avec un peu de trypsine. Lorsque les cellules sont décollées, l'action de la trypsine est bloquée par addition de milieu de culture contenant du sérum de veau foetal. Après centrifugation, le culot est repris par du milieu complet HAM F-12, puis les cellules sont comptées à l'aide d'un hemocytomètre de Malassez ou de Thoma. On prépare ensuite une solution à 27 500 cellules / ml dans du milieu de culture complet et on en dépose 200µl dans chacun des 60 puits centraux des microplaques de 96 puits.Il y a ainsi 5500 cellules par puits. Les plaques sont mises à incuber à 37 °C en atmosphère humide air/CO₂ (95% / 5%).Le test est effectué après 48 h lorsque les cellules adhèrent au support.
■ *Test*
   Les différentes fluoroquinolones (ou statines) sont utilisées en solution allant de 0.1 µM à 1 mM dans un solvant adapté dont l'innocuité a été préalablement vérifiée. La ténotoxicité de ces molécules a été étudiée selon différents protocoles: sans prétraitement ou avec prétraitement par l'anéthole-dithiolethione (à la dose de 20µM). 72 heures avant mise en contact avec les xénobiotiques; les cellules sont mises alors en culture en microplaques directement en suspension dans les solutions de l'anéthole-dithiolethione.
   Le jour du test, après avoir observé la confluence et l'état microscopique des cellules, on élimine le milieu de culture par retournement des plaques et on distribue dans chaque puits 200µl d'une solution 20 µMolaire de dihydro-dichlorofluoresceine diacetate (H₂DCF-DA) , sonde fluorogène qui pénètre dans les cellules et reste localisée dans leur cytosol; ce produit est utilisé en routine pour évaluer les radicaux libres produits par la cellule.
   Les plaques sont alors placées à l'étuve à 37° pendant 20 minutes.
   Après élimination de la sonde par retournement des plaques,les puits sont traités par les différentes solutions de xénobiotiques et mis à l'étuve . On place ensuite les plaques dans le fluorimètre équipé du filtre d'émission de 535 nm et du filtre d'excitation de 485 nm.
   Les unités de fluorescence sont lues après 40 minutes.
■ *Résultats*
   Les résultats sont exprimés en pourcentage de fluorescence par rapport au témoin.

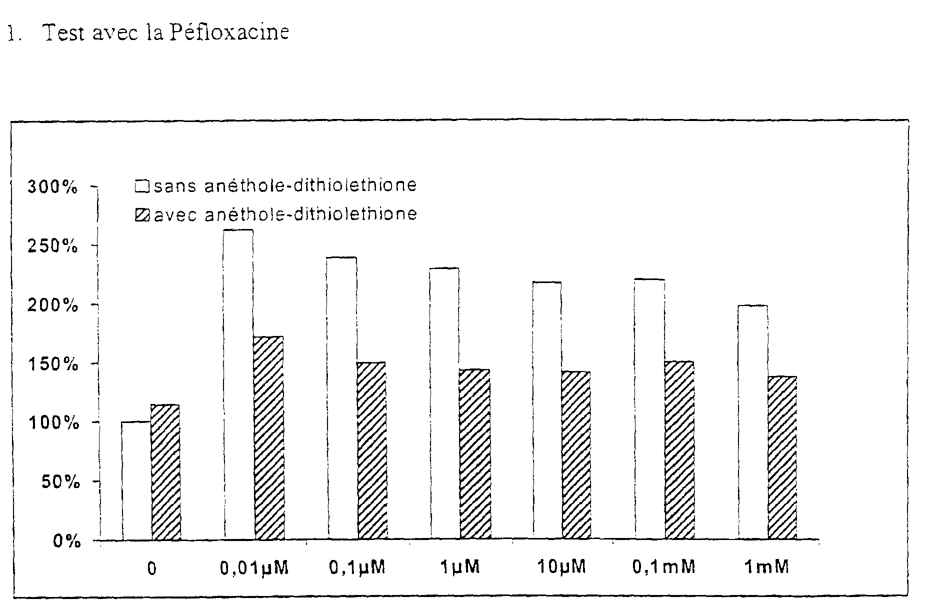

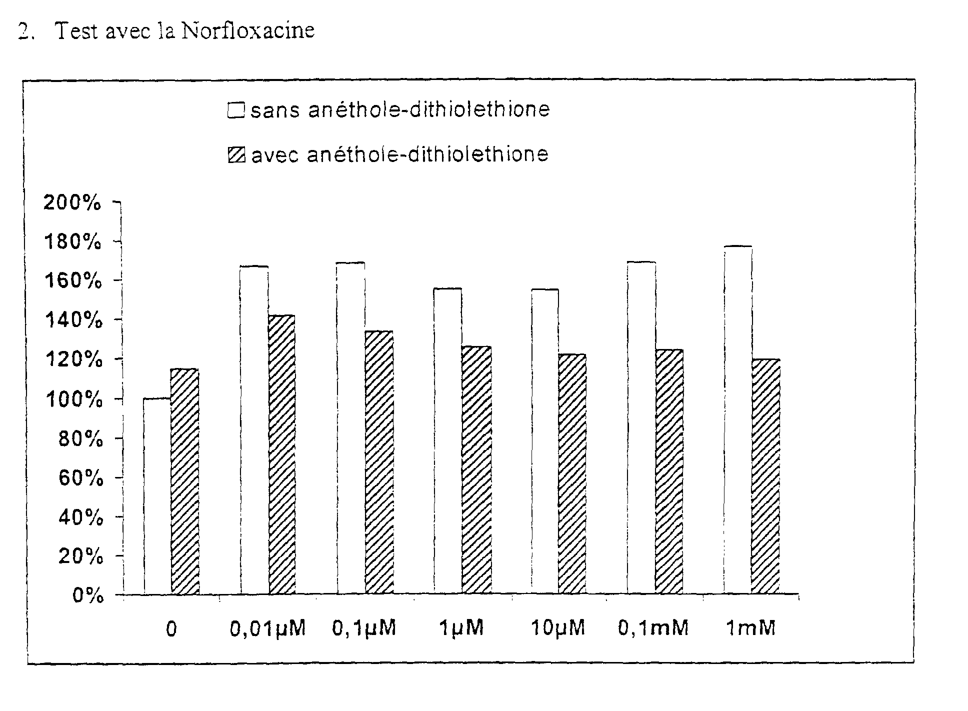

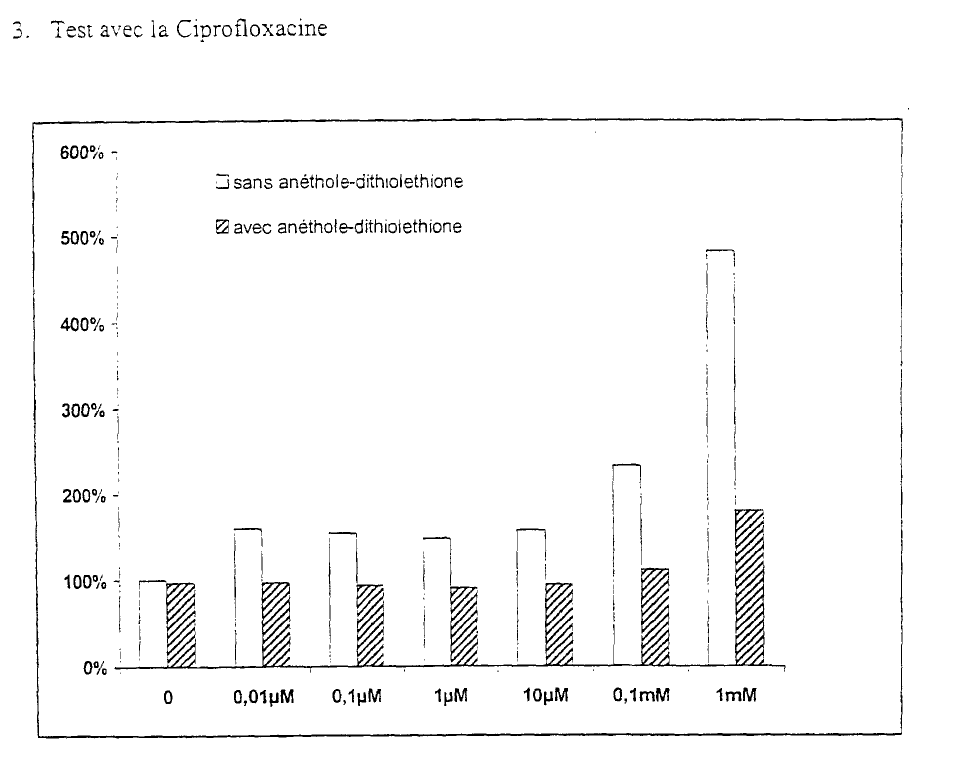

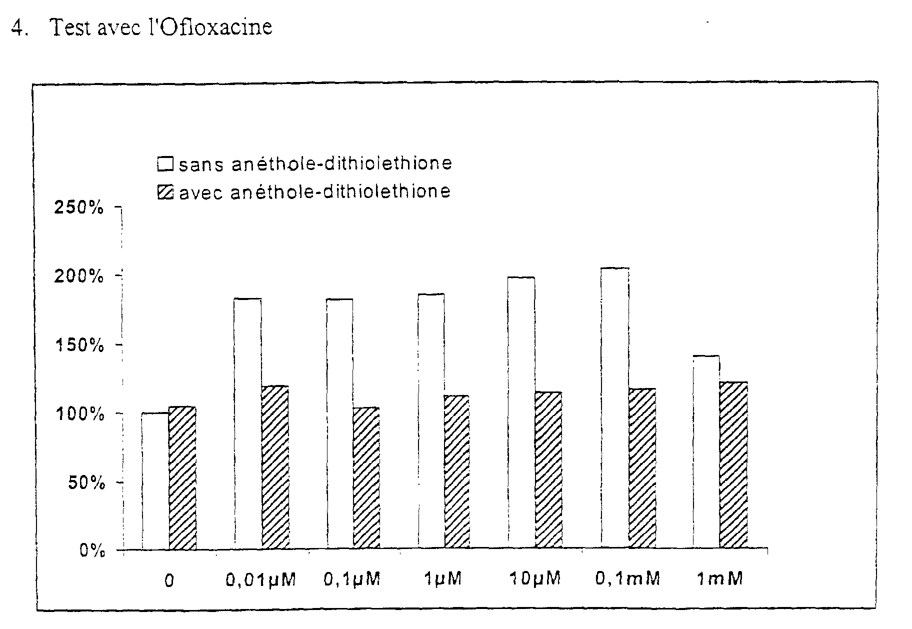

## Revendications

1. Utilisation de l'anéthole-dithiolethione pour la fabrication d'un médicament pour la prévention ou le traitement de la ténotoxicité induite par un médicament inducteur.

2. Utilisation selon la revendication 1, pour la fabrication d'un médicament contenant de 10 à 200 mg d' anéthole-dithiolethione.

## Patentansprüche

1. Verwendung von Anethol - dithioethion für die Herstellung eines Medikaments für die Prophylaxe oder die Behandlung einer von einem Induktionsmedikament hervorgerufenen Sehnentoxizität.

2. Verwendung gemäss Anspruch 1, für die Herstellung eines Medikaments enthaltend 10 bis 200mg Anethol - dithioethion.

## Claims

1. Use of anethole-dithiolethione in the manufacture of a drug for preventing or treating tendon toxicity induced by an inductive drug.

2. Use according to claim 1 in the manufacture of a drug containing from 10 to 200 mg of anethole-dithiolethione.
